Europäisches Patentamt

European Patent Office

Office européen des brevets

(11) Publication number: **0 485 701 A1**

# EUROPEAN PATENT APPLICATION

(21) Application number: **91114668.6**

(22) Date of filing: **30.08.91**

(51) Int. Cl.5: **C12N 15/63**, C12N 15/90, A61K 39/00, C12N 15/70, C12N 15/74, //C12N15/30, C12N15/31

The applicant has subsequently filed a sequence listing and declared, that it includes no new matter.

(30) Priority: **28.09.90 US 590364**

(43) Date of publication of application: **20.05.92 Bulletin 92/21**

(84) Designated Contracting States: **AT BE CH DE DK ES FR GB GR IT LI LU NL SE**

(71) Applicant: **AMERICAN CYANAMID COMPANY 1937 West Main Street P.O. Box 60 Stamford Connecticut 06904-0060(US)**

(72) Inventor: **Brey, Robert Newton, III 74 Sagamore Drive Rochester, New York 14617(US)** Inventor: **Deich, Robert Allen 10 Fallbrook Circle Rochester, New York 14625(US)**

(74) Representative: **Wächtershäuser, Günter, Dr. Tal 29 W-8000 München 2(DE)**

(54) **Insertion of DNA by modified transposons.**

(57) DNA constructs for the introduction of a DNA sequence into the constituent DNA of a prokaryote, and methods of use. The DNA construct includes an expressible gene encoding a transposase protein, linked in cis to a transposable cassette. The transposable cassete includes a pair of transposase recognition sequences flanking the DNA sequence. The gene encoding the transposase protein is not flanked by the transposase recognition sequences.

EP 0 485 701 A1

This is a continuation-in-part of copending application Serial Number 07/590,364, filed on September 28, 1990, the contents of which are incorporated herein by reference.

## BACKGROUND OF THE INVENTION

Prokaryotic transposable elements are discrete DNA sequences capable cf insertion at single or multiple sites within a prokaryotic genome (for a review see D.E. Berg ed., (1989) Mobile DNA, ASM Publications, Washington, D.C.). Historically, these transposable elements have been divided into 3 classes.

The first class comprises small transposable elements, generally less than 2,000 base pairs in length. These elements are called insertion sequences or IS sequences, and encode only determinants relevant to their own transposition; specifically, a transposase protein. This first class also includes composite transposons which are a segment of DNA flanked by two copies of an insertion sequence. The terminal portions of all IS sequences comprise inverted repeat sequences. The transposase protein functions by recognizing these terminal sequences and interacting with the sequences to effect transposition within the genome.

The second class of transposons is the Tn3 family of transposons. These transposons encode two products involved in a two-step transposition process; a transposase and a resolvase. Transposons belonging to this second class have inverted terminal repeats of approximately 35-40 base pairs.

The third class includes bacteriophage Mu and related phages. Bacteriophage Mu is large relative to other transposons with a genome of 36,000 base pairs. Mu encodes two gene products which are involved in the transposition process, a 70,000 dalton transposase and an accessory protein of approximately 33,000 daltons. An unusual feature of Mu that distinguishes it from other transposons is that its ends are not inverted repeat sequences. The Mu transposase has, however, been shown to bind to both ends in an in vitro binding assay.

The use of transposons as vehicles for the introduction of foreign DNA into a host cell chromosome has been described previously (e.g., Way et al., Gene 32:369-379 (1984)). Also, Grinter (U.S. Patent No. 4,784,956) describes a two-vector method for inserting a transposable cloning vector into the chromosome of a recipient cell. The first plasmid carries a transposon having the gene encoding its transposase function replaced with foreign DNA to be inserted into the chromosome of the recipient cell. The second plasmid carries an expressible transposase gene. The transposase function is, therefore, provided in trans.

Narang et al. (U.S. Patent No. 4,830,965) describe a construct referred to as a transposable linker. The linker comprises the extreme ends, or transposase recognition sequences of a transposon. The genetic material which is flanked by the transposase recognition sequences in the naturally occurring transposon has been replaced with a DNA sequence containing restriction enzyme recognition sequences to facilitate the insertion of a foreign DNA sequence. As was the case with the Grinter method, Narang et al. teaches the introduction of a gene encoding a transposase protein on a separate plasmid vector, in trans.

## Summary of the Invention

This invention pertains to a DNA construct for the introduction of a DNA sequence into the constituent DNA of a prokaryotic cell. The DNA construct comprises a gene encoding a transposase protein linked, in cis, to a transposable cassette. The transposable cassette comprises a pair of transposase recognition sites (usually inverted repeats) flanking a cloning site for the insertion of the DNA sequence to be introduced into the constituent DNA of the prokaryotic cell.

The invention can be used, for example, to insert single or multiple copies of an expressible gene into the chromosome of a prokaryotic host. This enables the efficient production of antigenic material for vaccine applications. Furthermore, if the prokaryotic host is an attenuated enteroinvasive bacterium, the invention is useful for the construction of a strain useful in a live vaccine formulation.

Providing the transposase function in cis (i.e. linked on a continuous DNA molecule) offers many advantages over known techniques in which the function is provided in trans. For example, if the DNA sequence to be introduced is carried on a separate vector from the gene encoding the transposase protein, then co-transformation of a cell with both vectors is necessary to achieve the desired result. The construct of this invention enables the introduction of a transposable sequence by transforming a cell with a single vector. This is a more efficient and convenient methodology because it reduces the number of cells which must be screened in order to identify a clone having the desired properties.

Another advantage relates specifically to the IS transposons. The IS transposases have been shown to be inefficient when introduced in trans. Complementation in trans of an IS transposon with a defective transposase occurs with an efficiency of about 1% or less (see e.g., Morisato et al., Cell 32:799-807 (1983)).

One explanation which has been advanced for this phenomenon is that the IS encoded transposases bind nonspecifically to DNA sequences which are physically close to the transposase gene immediately upon synthesis. These nonspecifically bound molecules then scan the DNA in search of transposase recognition sequences by one-dimensional diffusion along the DNA (Berg et al., (1981) Biochemistry 20:6929-6948). In contrast, the DNA constructs of the present invention encode the transposase gene and the transposase recognition sequences in cis, resulting in an efficient transposition system.

Brief Description of the Drawings

Figure 1 is a diagram representing a DNA construct of this invention.

Figure 2 is a diagram representing steps in the construction of a DNA construct of this invention.

Figure 3 is a diagram representing (A) the DNA sequence of a synthetic oligonucleotide specifying the left end of the prokaryotic transposon TnIO and (B) the DNA sequence of a synthetic cloning linker for use with synthetic transposase recognition sequences.

Figure 4 is a diagram representing two DNA constructs of this invention which are derived from bacteriophage λ.

Figure 5 is a diagram representing a transposon exchange reaction between a plasmid and a bacteriophage.

Figure 6 is a diagram representing steps in the construction of a bacteriophage based DNA construct of this invention which carries an expressible Plasmodium berghei circumsporozoite gene.

Figure 7 is a diagram representing steps in the construction of a plasmid based genetic construct of this invention which carries an expressible LT-B gene.

Figure 8 is a diagram representing schematically chromosomal transposon insertion in Salmonella.

Figure 9 is a diagram representing a synthetic oligonucleotide linker specifying restriction sites unique in bacteriophage λ.

Figure 10 is a diagram representing the results of electroporation of pPX1575 into H. influenzae HDG85.

Detailed Description of the Invention

The subject invention relates to DNA constructs for the introduction of a DNA sequence into the constituent DNA of a prokaryotic cell, and to methods for their use. The constituent DNA of the prokaryotic cell includes all autonomously replicating DNA molecules within the cell including, for example, chromosomes and episomes. Transposition is often independent of the constituent DNA sequence of the prokaryotic cell and therefore transposition generally occurs at random locations. However, transposons can also, with a high frequency, exchange with other transposons of the same type via transposition or double recombination between tranposase recognition sequences. As discussed below, this exchange mechanism facilitates the insertion of a transposon carrying, for example, a gene of interest at a specific location in the constituent DNA of a host cell.

This invention can be used to introduce a DNA sequence having or encoding any function. For example, the DNA sequence can comprise a sequence encoding a structural or regulatory protein, or can comprise a regulatory sequence such as a promoter. Alternatively, the inserted sequence can be one not known to possess any biological function which can be used to interrupt a cellular function, for example, by inserting itself within an essential gene thereby interrupting the function of the gene.

In preferred embodiments, the DNA sequence to be introduced into the constituent DNA of a prokaryotic cell is an expressible gene. The expressible gene includes all necessary regulatory sequences including, for example, a prokaryotic promoter, ribosome binding site, etc.

The invention describes basic methodology to insert single or multiple copies of genes into chromosomes of diverse species. Expressible genes can be derived from eukaryotic sources and can encode antigenic components from pathogenic parasites, human immunoactive proteins and peptides, hormones, growth factors, allergens, tumor associated antigens and other proteins. Such genes can be derived from viral sources and can encode antigenic proteins, structural components, or enzymes involved with viral replication or attachment. Homologous genes as well as heterologous genes can be derived from bacterial, viral, parasite, fungal or mammalian sources and may include genes encoding virulence factors of pathogenic organisms, including toxins, protective immunogenic proteins, or genes encoding proteins involved in the regulation or synthesis of antigenic polysaccharide material, and in addition can be enzymes foreign to the host bacterium.

3

Among the bacterial antigens of interest are those associated with the human bacterial pathogens including, for example, typable and untypable Haemophilus influenzae, Escherichia coli. Neisseria meningitidis, Streptococcus pneumoniae, Streptococcus pyogenes, Branhamella catarrhalis, Vibrio cholerae, Corynebacterium diphtheriae, Chlamydia trachomatis, Neisseria gonorrhoeae, Bordetella pertussis, Pseudomonas aeruginosa, Staphylococcus aureus, Streptococcus pyogenes, Klebsiella pneumoniae, and Clostridium tetani. Examples of specific bacterial antigens of interest include bacterial proteins of which particularly useful examples are outer membrane proteins (e.g. from Haemophilus influenzae, Branhamella catarrhalis or Neisseria meningitidis), bacterial toxins, (e.g., pertussis toxins, diphtheria toxin, tetanus toxin, and Pseudomonas exotoxin A), and bacterial surface proteins (e.g., flagellins, hemagglutinins and the M protein from Streptococcus pyogenes).

Viral antigens from pathogenic viruses include but are not limited to, human immunodeficiency virus (types I and II), human T lymphocyte virus (types I, II and III), respiratory syncytial virus, hepatitis A, hepatitis B, hepatitis C, non-A and non-B hepatitis viruses, herpes simplex virus (types I and II), cytomegalovirus, influenza virus, parainfluenza virus, poliovirus, rotavirus, coronavirus, rubella virus, measles virus, varicella, Epstein Barr virus, adenovirus, papilloma virus and yellow fever virus.

Several specific viral antigens of these pathogenic viruses include the F (peptide 283-315) and G proteins of respiratory syncytial virus (RSV), VP4 polypeptide of rotavirus, VP7 polypeptide of rotavirus, envelope glycoproteins of human immunodeficiency virus and the S and pre-S antigens of hepatitis B.

Genes encoding antigens of parasites and fungi can also be used in the present invention. Parasite antigens of interest include, for example, those of Plasmodium spp., Toxoplasma spp., Leishmania spp., Trypanosoma spp., Schistosoma spp., Toxocara spp., Ascaris spp., and Fasciola spp. Fungal antigens include those of Candid albicans, Cryptococcus neoformans and Coccidiodes immitis.

Also of interest are various antigens associated with auto-immune diseases, such as rheumatoid arthritis and lupus erythematosus, tumor antigens, cancer antigens and single or multiple copies of genes encoding hormones, bioactive peptides, cytokines, lymphokines and growth factors, as well as enzymes and structural proteins of prokaryotic or eukaryotic origin, especially for use in vaccines and therapeutics.

To provide novel vaccine formulations, genes encoding protective antigens can be introduced into the chromosomes of attenuated bacteria, which act as delivery vehicles for stimulation of immune responses against the pathogen from which the expressed gene was derived. See Dougan and Tite, Seminars in Virology 1:29 (1990). Genes encoding antigens derived from pathogenic bacterial, viral, or parasitic sources can be introduced into chromosomes of attenuated S. typhi for use as live vaccines for use in humans (to protect against, for example, typhoid fever, diarrheal diseases, and sexually transmitted diseases including AIDS). Alternatively, such genes can be integrated into chromosomes of other Salmonella capable of infecting animal species, e.g., S. dublin for use as live attenuated cattle vaccines (e.g., against shipping fever), S. choleraesuis for use as live attenuated vaccines for swine, and S. gallinarum or S. pullorum for use as live attenuated vaccines for poultry. In a particular embodiment, antigens derived from Eimeria parasites can be inserted into the chromosome of attenuated S. gallinarum to produce an oral vaccine for coccidial disease. Alternatively, genes encoding antigens can be inserted into chromosomes of other bacteria to be used as live vaccines. Examples of such can include attenuated enteroinvasive Escherichia coli, Yersinia enterocolitica, Shigella flexneri, S. dysenteriae, Campylobacterium jejuni, Vibrio cholera, and Bacille Calmette-Guerin (BCG), an avirulent mutant of Mycobacterium bovis.

In addition, such genes can be inserted as single or multiple copies into bacterial chromosomes to provide for efficient production of antigenic material for vaccine production. Insertion of native or mutated genes derived from bacterial pathogens into chromosomes can result in enhanced and efficient antigen production. For example, genes encoding mutant toxins, genes encoding virulence factors, or other native or mutant protective immunogens can be introduced into the chromosome of a homologous or heterologous bacterial strain. In many cases, virulence factors or protective immunogens can be produced efficiently only in a particular host bacterium. In addition, genes which encode products involved in complex biosynthetic pathways leading to bacterial surface polysaccharide or capsule production are too large to be cloned and expressed in other bacteria for the purpose of producing antigenic material for vaccine uses. Examples of such uses include the insertion of cross-reacting mutated toxin molecules of Bordetella pertussis into the chromosome of toxin-deficient organisms for the production of an efficient pertussis vaccine and the insertion of altered regulatory signals enhancing the production of a mutant toxin gene product.

Antigenic capsular material derived from Haemophilus influenzae type b can be obtained from fermentation of the bacteria. Insertion of altered genes affecting regulation of capsule synthesis can lead to enhanced production of capsular material in H. influenzae. Efficient production of immunogenic outer membrane proteins of H. influenzae can be enhanced by insertion of genes encoding novel fusion proteins in a genetic background devoid of wild type outer membrane protein activity.

4

Expressible genes can be inserted into chromosomes resulting in recombinant organisms capable of high level production of protein products from a single gene copy. By random insertion of genes into chromosomal loci, bacteria expressing high levels of gene products can be isolated. Random localization leads to isolates in which positional effects enhancing gene expression can be detected. Enhanced gene expression can yield stable recombinants which express protein at levels exceeding similar gene constructs carried on multiple copy plasmid vehicles.

Expressible genes can be inserted into the chromosome so that inheritance of the inserted genes can be maintained indefinitely in a stable fashion. Extrachromosomal plasmid vectors which are commonly used for expression of foreign proteins can be unstable leading to loss of foreign gene expression. Insertion of genes into random or specific locations within bacterial chromosomes leads to stable inheritance and retention of gene expression.

It is possible to enrich for and to screen for stable insertion of multiple copies of the same gene in the chromosome of a bacterial strain. This invention provides for introduction of multiple copies of the same gene in the chromosome of the host organism or one to several copies of different genes.

The DNA construct of this invention comprises a gene encoding a transposase protein linked in cis to a transposable genetic cassette as a single continuous DNA molecule. The cassette comprises a pair of transposase recognition sites (usually inverted repeats) flanking a cloning site for insertion of the DNA sequence to be introduced into the constituent DNA of the prokaryotic cell, but not flanking the gene encoding the transposase protein.

Naturally occurring transposable elements have transposase recognition sites occurring at their 5' and 3' termini. These ends and any internal DNA sequence can transpose throughout the constituent DNA of a cell. This internal sequence can vary greatly in length from approximately 1,500 base pairs in the case of IS sequences to much longer sequences in the case of phage Mu. A common feature of this internal sequence in all transposons is that a portion of it encodes a transposase which specifically recognizes the 5' and 3' terminal sequences, or transposase recognition sequences, of the particular transposon in which it is contained.

In contrast to the naturally occurring situation described above, the construct of this invention carries a transposable cassette having transposase recognition sequences, but these recognition sequences do not flank the transposase gene. The transposase gene has been inserted at another location in the construct of this invention. The transposed sequence does not include a transposase gene and is, thus, stably integrated in the absence of any external transposase gene. The methods of this invention enable one to stabilize a particular cloned prokaryotic cell having a transposable cassette contained within its constituent DNA at a particular location.

Any set of transposase recognition sequences, and genes encoding the corresponding transposase can be used to practice this invention. In a preferred embodiment, the transposase recognition sequences comprise inverted repeats. Naturally occurring transposons having inverted repeats include, for example, ISI, IS2, IS3, IS4 and IS5; and the Tn elements including, for example, Tn1, Tn2, Tn3, Tn5, Tn9, TnIO and Tn903. The transposase recognition sequences of bacterophage Mu and related transposons, are also useful for the practice of this invention. The transposase recognition sequences of phage Mu and related transposons are not inverted repeats. Such sequences, and sequences encoding the corresponding transposase protein, can be isolated from the naturally occurring transposon using standard techniques. Alternatively, such sequences, or sequences which are functionally equivalent and substantially homologous, can be synthesized chemically.

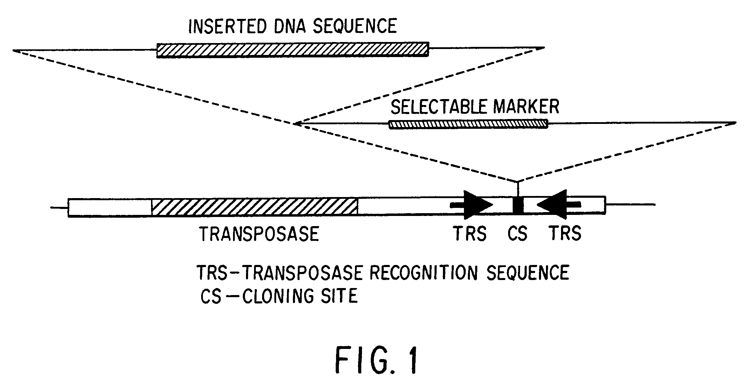

The construct of this invention is synthesized through a series of genetic manipulations employing methods and enzymes known to those skilled in the art. The two essential elements are the transposable cassette and the expressible transposase gene. These elements are linked in cis to form a single continuous DNA molecule. Preferably, as shown in Figure 1, this single continuous molecule is linked to an origin of replication which enables the conditional propagation of the construct in at least one type of prokaryotic cell, and a selectable marker such as a gene encoding a protein conferring antibiotic resistance, the selectable marker being flanked by the transposase recognition sequence.

Although selectable markers which lead to antibiotic-resistance can be readily used to construct the above described modified transposable elements, drug-resistant organisms are not suitable for all purposes, especially for use in live bacterial vaccine applications, where dissemination of the drug-resistance allele to other gut flora is possible. During fermentation for production of recombinant proteins, enzymes and other products, addition of antibiotics to growth medium allows for selection for cells containing plasmids or modified transposable elements, but can be unacceptable in many instances because of cost and possible

contamination of the endproduot. For the modified transposons described here, antibiotic-resistance is used to select for insertion, and can be eliminated during subsequent steps because of the inherent stability of the elements.

To replace the antibiotic-resistance determinants with selectable markers which could be used where dissemination of the organisms in the environment is possible, there are numerous strategies which can be applied. The modified transposons can be selected and introduced into recipient cells by complementation of host chromosomal mutations. Complementation systems rely on construction of particular host chromosomal mutations, but can be used reliably to circumvent the inclusion of antibiotics to the culture medium. Selectable markers that have been used in some instances to select and maintain plasmids in bacterial cultures during fermentation can be used to select transposition events. For example, complementation of a chromosomal mutation for aspartic semialdehyde dehydrogenase (asd) in Salmonella typhimurium or D-alanine racemase mutation (dal) in Bacillus subtilis, which each lead to faulty cell wall biosynthesis and cell lysis, yields stable plasmid inheritance in the absence of selection in all viable cells of the culture. Both the asd and dal mutations can be supplemented with nutritional additives. Thus, transposons marked with a functional dal or asd gene can be selected in bacterial hosts containing those mutations. On the other hand, complementation of an essential gene of the host, which cannot be provided by nutritional supplementation can also lead to stable plasmid maintenance. An E. coli gene, ssb, is required for DNA replication and cell viability, which prevents the accumulation of plasmidless cells during fermentation in a bioreactor when placed on a plasmid. Analogously, a plasmid-borne copy of valyl tRNA synthetase stabilizes plasmids in E. coli containing a chromosomal temperature-sensitive valyl tRNA synthetase. Plasmids can also be stabilized by inclusion of a bacteriophage repressor gene, the loss of which leads to induction of host prophage and cell death. So, functionally any of those selection systems can be applied to transposable elements described above. In addition, transposable elements can be modified to contain selectable markers which can be used in bacterial cell recipients which do not have to be modified by mutation to accept the marker. For instance, genes for resistance to heavy metals (Silver, S. and Misra, T.K., Ann. Rev. Microbiol., 1988, 42:717-743. Plasmid mediated heavy metal resistance) which are not used for human therapy can be introduced into the transposable elements. These markers would include genes which encode resistance to arsenite or arsenate, antimony, cadmium or organo-mercurial compounds. In a particular embodiment, the arsA and arsB genes, which are solely involved with resistance to arsenite, can be easily adapted to the modified transposable elements.

Also useful as selectable markers are genes encoding resistance to herbicides. A number of such genes have been described, each conferring resistance to a limited range of herbicides, such as imidazolinones, sulfonylureas (Yadav et al., PNAS USA 83:4418-22, 1986) glyphosate (Comai et al., J. Biol. Chem. 260:4724-4728, 1985); and phosphothrinicin and bialophos (Thompson et al., EMBO J 6:2519-2523, 1987; DasSarma et al., Science 232:1242-1244, 1986).

For particular application to attenuated Salmonella live vaccine applications, modified transposons can be marked with a gene which complements a secondary attenuating locus to insure vaccination of the host mammal with antigen-expressing organisms. For example, the gene for adenyl succinate synthetase, encoded by the purA gene, can be introduced into the modified transposons also containing an expressed antigen. Transposition events can be selected in Salmonella vaccine strains by selecting for adenine independence in a purA genetic background. When a purA mutation is present in conjunction with other attenuating mutations, such as aroA, Salmonella cells are essentially hyper-attenuated and do not replicate in the mammalian host. Thus, vaccine strains which contain a purA-complementing transposon are fully immunogenic.

One skilled in the art can synthesize a genetic construct as described herein using well known molecular cloning techniques. Furthermore, sources for each of the necessary starting components are generally available. For example, a gene encoding a selectable marker such as Kan[R] can be excised from any of a number of common sources (e.g., Tn5) by digestion with appropriate restriction enzymes. Sources for transposase recognition sequences and the corresponding transposon gene have been described above.

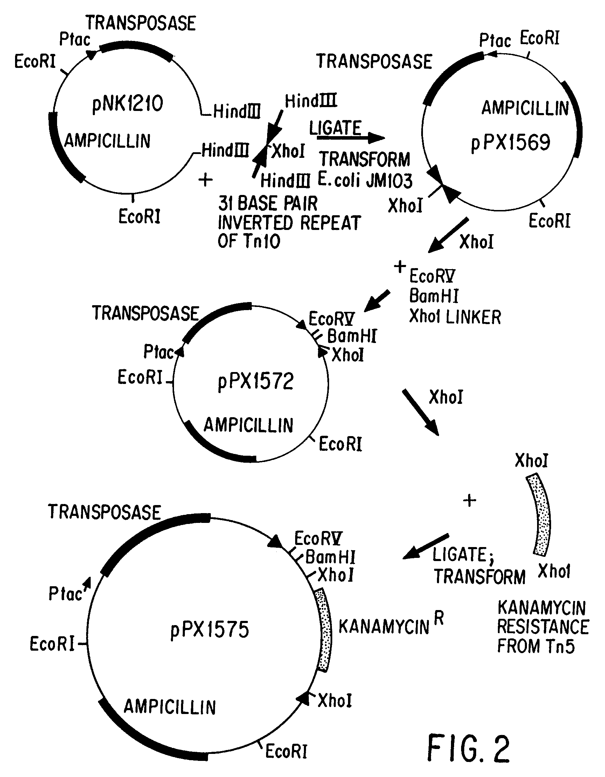

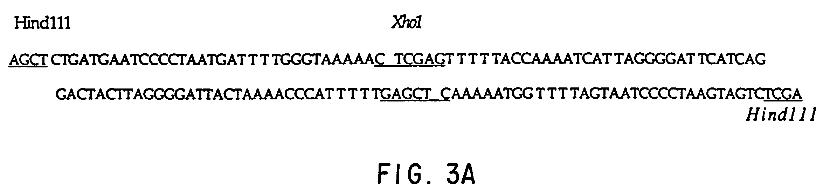

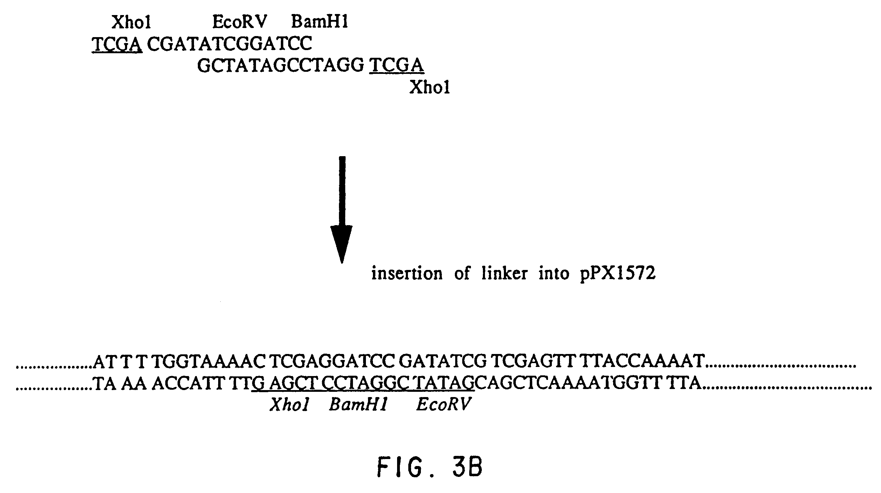

Figure 3A represents a synthetic oligonucleotide specifying the extreme 31 base pairs of the right end of Tnlo; approximately 27 base pairs of Tn 10 is sufficient for recognition by the Tn 10 transposase (Way and Kleckner, Proc. Natl. Acad. Sci. USA 81:3452-3456 (1984)). Cloning sites for insertion of the DNA sequence (preferably a multiple cloning site containing several unique restriction enzyme recognition sequences) are also widely available or can be synthesized chemically. Figure 3B, for example, represents a synthetic cloning linker which was inserted into pPX1569 to generate pPX1572, as shown in Figure 2. The order and arrangement of the components is clearly provided in Figures 1 and 2. One skilled in the art will recognize that the positions of the transposase in the DNA construct is not critical provided that it is expressible, and outside of (i.e., not flanked by) the transposase recognition sequences (generally inverted repeats). Similarly, the order of the selectable marker and the inserted DNA sequence can be the reverse of that shown in Figure 1, provided that both are flanked by the transposase recognition sequences. In a preferred embodiment, each of the above-identified components are positioned in a replicon containing vector DNA molecule. Any cloning vector, modified as described above, is appropriate.

The construct described above can be propagated by transforming a prokaryotic cell in which the replicon is functional. The replicated construct can then used in further steps of genetic manipulation. For example, using well known techniques any sequence (e.g., a gene of interest), can be inserted into the cloning site of the transposable cassette and the new construct can be used to transform prokaryotic cells.

In the transformed cell the transposase gene will be transcribed, as it carries all of the necessary regulatory sequences (e.g. promoter, ribosome binding site, etc.). The transposase protein recognizes the ends of the transposable cassette thereby inducing its transposition into the constituent DNA of the cell. The gene carried within the genetic cassette, which also has the necessary regulatory signals, will also be expressed.

In preferred embodiments, the replicon is conditional. A conditional replicon replicates under permissive conditions, but not under restrictive conditions. The use of conditional replicons enables the stabilization of a particular cloned prokaryotic cell having a transposable cassette contained within its constituent DNA at a particular location. This stabilization is accomplished by removing or effectively inactivating the transposase gene (by growing the cell under restrictive conditions), and ultimately, the transposase protein, from the cloned cell.

For example, some plasmids carry a conditional defect, such as a temperature-sensitive replicon. Constructs of this invention which are derived from such plasmids have the ability to replicate at a permissive temperature, but cannot replicate at a restrictive temperature. Cells carrying the plasmid can, however, grow and divide at both temperatures. After a cell having a transposable cassette contained within its constituent DNA at a particular location is identified, the incubation temperature is shifted to the restrictive temperature. The cells continue to grow and divide but the construct does not replicate. The gene construct carrying the transposase gene, and consequently the transposase protein, is thereby diluted to the extent that the integrated transposable cassette is stabilized within the constituent DNA. An example of such a curable plasmid is the temperature sensitive E. coli F factor. Any replicon having such a conditional defect is useful in the practice of this invention.

Limited host-range plasmids are conditional and can also be used to enable the stabilization of a desired integrate. Well known examples of this group of plasmids are derivatives of the E. coli colEl plasmid vector pBR322. These plasmids are small and easy to prepare in large quantities for in vitro cloning steps. They are limited in host-range to E. coli and closely related enterobacteria, and are replication incompetent in other species unless the E. coli DNA polymerase I function is provided. This dependence is due to the requirement of the colEl origin of replication for the E. coli-like DNA polymerase I function. Hence, these plasmids can be used as suicide delivery vectors for other bacteria. Suicide delivery, as used herein, refers to the introduction of a transposon via a genetic construct which cannot replicate in the host. The plasmids direct the synthesis of the transposase protein upon transformation which results in the integration of the transposable cassette into the constituent DNA. However, because the replicon is replication incompetent in the prokaryotic cell selected, the tranposase protein is quickly diluted to the point where transposition no longer takes place.

Any plasmid vector which has limited host range can be a delivery vehicle for the modified transposons described above. For instance, the origin of pBR327, used in construction of pPX1575 and its derivatives, freely replicates in E. coli and Salmonella, but fails to replicate in H. influenzae or B. pertussis. Thus, pPX1575 is a suicide plasmid for delivery of modified transposons to those bacterial cells. Conditional replicons for delivery of modified transposon to enteric bacterial hosts can be constructed as an alternative to delivery in bacteriophage such as lambda or temperature-sensitive F factors as described above. The origin of plasmid R6K requires a functional $\pi$ protein, which functions in trans for replication. Suicide vectors containing the R6K replicon have been constructed (e.g., Miller, V.L. and Mekalanos, J.J. 1988, J. Bacteriol. 170:2575-2583). Using E. coli hosts which contain the $\pi$ protein in trans, suicide plasmids carrying the modified transposable elements can be isolated. These suicide plasmids would act to deliver transposable elements, by transformation or by conjugation, to such bacteria as Salmonella, Shigella, and other closely-related enteric bacteria. Further, replicons derived from gram-positive organisms, such as pUB110, originally isolated in S. aureus, fail to replicate in gram-negative bacteria such as E. coli or Salmonella, and thus can be used in construction of suicide plasmid vehicles for modified transposons.

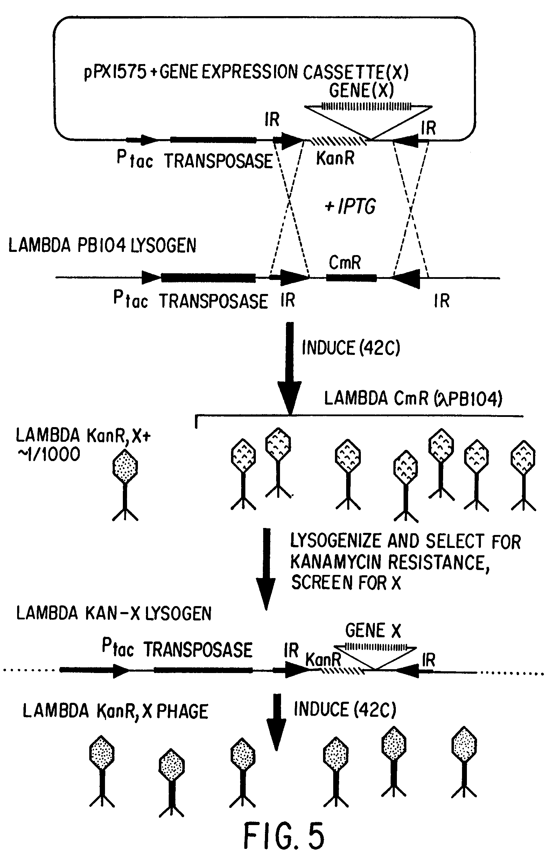

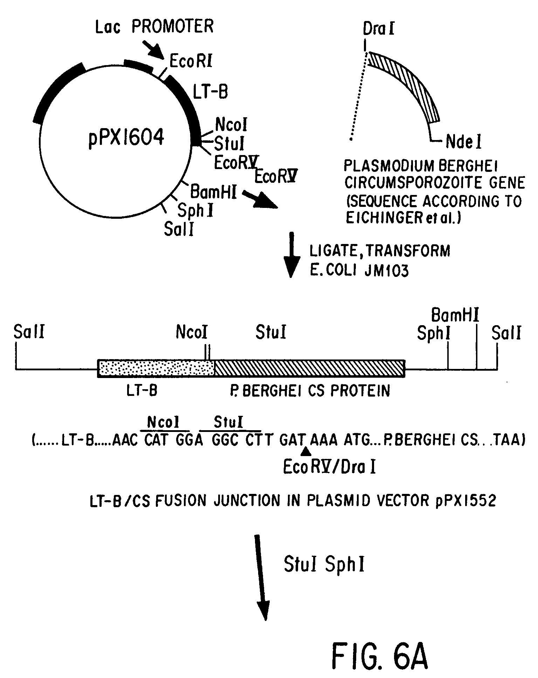

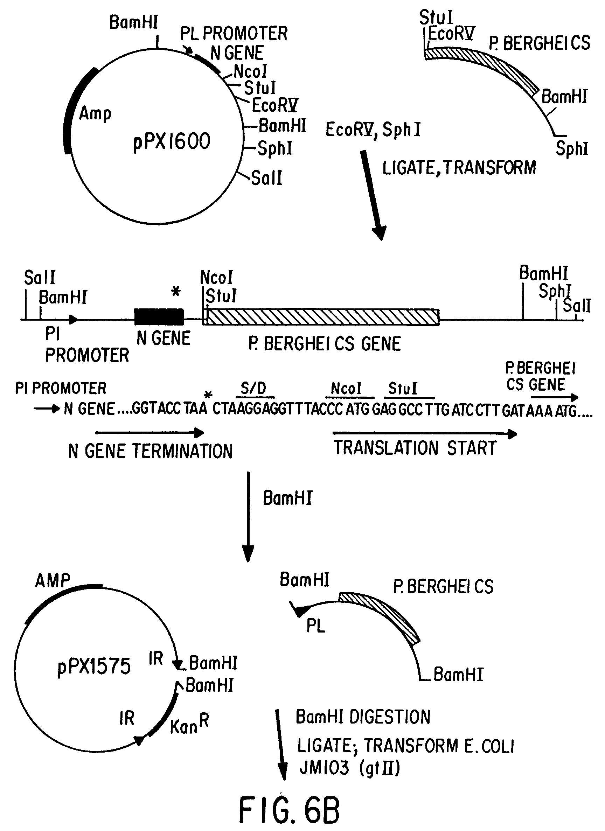

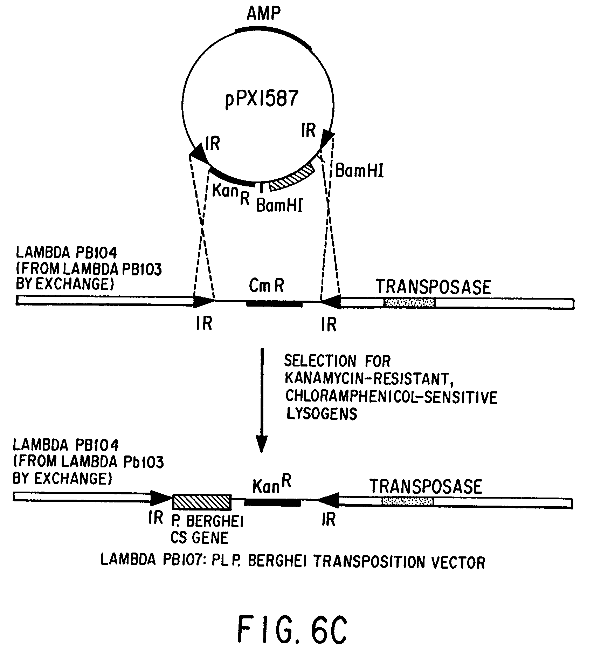

A third type of DNA construct for delivery of the transposable cassette in a prokaryotic chromosome is a bacteriophage derivative. In a preferred embodiment, the bacteriophage is bacteriophage $\lambda$. As shown in Figures 5 and 6, a phage which is unable to replicate in the prokaryotic host cell (i.e., a conditional replicon)

7

is used to deliver the transposable cassette. The system takes advantage of the property of resident transposons to spontaneously exchange in vivo. Hence, if construction of a desired specialized transposon is carried out on a plasmid carrying a first selectable marker is introduced into an E. coli cell lysogenized with a λ phage containing the suitable transposase recognition sequence flanking a second selectable marker, the plasmid transposon can exchange with the prophage transposon at a high frequency. Such exchanges can easily be detected and isolated by inducing the resident prophage and screening the lysate for phage capable of transducing the first marker. These lysogens can then be induced to yield a pure population of transducing phage carrying the transposable genetic cassette. Another method for stabilizing a particular chromosomal insertion involves the use of a regulatable promoter to control expression of the transposase protein. Many regulatable promoters are known to those skilled in the art including tac, lac, recA, $P_L$, and T7. Such promoters are active under defined conditions, but inactive under other conditions.

The preceding discussion relates primarily to random insertion of a DNA sequence. However, by using a host cell containing a resident transposon at a predetermined genetic locus, it is possible to direct the insertion of a DNA sequence to that predetermined locus. This is due to specific transposition or homologous recombination between transposase recognition sequences (generally inverted repeats).

To determine the position of a resident transposon, or a DNA sequence introduced by the methods of this invention, is a relatively simple matter which can be accomplished by restriction enzyme digestion and gel analysis. In complex situations, Southern hybridization may be necessary. When a resident transposon is identified at a desired location, a genetic construct of this invention is introduced using any appropriate method. Homologous recombination will occur at some frequency and those cells in which recombination has occurred can be detected, for example, by Southern hybridization.

The invention is further illustrated by the following Examples.

## EXAMPLES

### Methods

#### (a) Bacterial strains and bacteriophage

The following abbreviations are used throughout this application when referring to drug resistance:

| | |
|---|---|
| kanamycin resistance | $Kan^R$ |
| kanamycin resistant | $Kan^R$ |
| kanamycin sensitive | $Kan^S$ |
| ampicillin resistance | $Amp^R$ |
| ampicillin resistant | $Amp^R$ |
| ampicillin sensitive | $Amp^S$ |
| chloramphenicol resistance | $Cm^R$ |
| chloramphenicol resistant | $Cm^R$ |
| chloramphenicol sensitive | $Cm^S$ |
| tetracycline resistance | $Tet^R$ |
| tetracycline resistant | $Tet^R$ |
| tetracycline sensitive | $Tet^S$ |

The following strains were deposited with the NRRL, Peoria, Illinois, on July 12, 1990, under the terms of the Budapest Treaty:

E. coli JM103/pPX1575.........accession #NRRL B-18672

E. coli MC1061/pMC9, λPBIOO...accession #NRRL B-18673

E. coli MC1061/pMC9, λPB103...accession #NRRL B-18674

E. coli C600/F'$_{ts}$lac::TnIO....accession #NRRL B-18675

E. coli JC1553/F112 was obtained from Dr. B. Bachmann (Coli Genetic Stock Center, Yale University) and has the genotype: argG6, metBl, his-1, leu6, recA7, mt12, xy17, ga16, lacYl, rpsL104, tonA2, tsx, $λ^R$, $λ^-$, supF44. F112 carries an intact λ receptor (lamB) and genes for maltose utilization ($Mal^+$).

Other E. coli strains used in construction of plasmids and recombinant λ phage are as follows: Y1088 (ΔlacU169 supE supF hsdR hsdM$^+$ metB trpR tonA21 proC::Tn5 (pMC9) [ATCC 37195]) was used as host strain in recovering recombinant λ lysogens (Young and Davis, Science 222:778 (1983)); MC1061 [ATCC 53338] (Δara-leu) 7697 Δ(lacPOZY) X74 hsdR hsdM$^+$ araD139 galE15 galKi6 rpsL) was used also as a host for some of the recombinant λ phage and for titering λ stocks (Meissner et al., Proc. Natl. Acad. Sci. USA 84:4171 (1987)); JM103 [ATCC 39403] (F'traD36 proAB$^{+1}$ laci$^Q$ ΔM15/Δ(pro-lac) supE hsdR sbcB15 thi-l endAl $λ^-$) was used as a host strain for construction of plasmids containing tac or lac-promoter regulated genes (Messing et al., Nucl. Acids Res. 9:309 (1981)); N99cI[+] (F[-]galK2 rpsL λ[-] cI[+]) is a host strain containing the wild type cI repressor gene, but heavily deleted for other λ functions (Gottesman et al., J. Mol. Biol. 140:57 (1980)); C600 [ATCC 47024] (F[-]thi leuB6 lacYl thr-l hsdR supE44 tonA21 λ[-]) was used as a host strain for the temperature sensitive F factor, F$_{ts}$lac TnlO; and KBTOOl (purE lacY lysA leuA, proC metE arqH rpsL λ[-]) was used as described in the Examples (Kadner and Liggins, J. Bact. 115:514-521 (1973)).

The bacteriophage λ cloning vehicle λgtII [ATCC 37194] (cI857 nin5 lac5 Sam 100 Δshnd 111 λ2-3 srlλ3° srlλ4° srlλ5°) was maintained in Y1089 (Young and Davis, Proc. Natl. Acad. Sci. USA 80:1194 (1983)). The λ cloning vehicle λNM816 (imm[21] clts nin5 lac5) was maintained as a lysogen in λ1089 (Murray, N., In:LAMBDA II (1983), Hendrix et al., eds., Cold Spring Harbor Laboratory, NY, p. 395).

Attenuated Salmonella vaccine strains used were: Ty523 (aroA Δ407 hisG46), a derivative of S. typhi CDC 1080 [U.S. Patent No. 4,735,801]; SL3261 (aroA554::TnlOTc[5] a stable non-reverting aroA deletion derived from S. typhimurium, biotype Wray; and SL1438 [ATCC 39184] (hisG46 aroA 554 TnlO Tc[S]), an aroA deletion mutant of S. dublin.

S. typhimurium LB5010 is a galE mutant derived from LB5000 (Bullas & Ryu, J. Bacteriol. 156:471 (1983)). The lamB receptor of E. coli was introduced into LB5010 by mating with E. coli KL188 containing F112dTnlO(cm), selecting for Cm[R]. The Cm[R] F112 was obtained by transforming F112/JC1553 with plasmid pNK1210 (Way et al., Gene 32:369-379 (1984)), and selecting Cm[R] exconjugants of PR13 (pnp13, rna19, thrl, leuB6, thil, lacYl, xy17, mt12, malA, rpsL, malB:described by Hautala et al., (Proc. Natl. Acad. Sci., 76:5774-5778 (1979)) which were sensitive to λ. The marked lamB[+] F prime factor was moved into a multiply auxotrophic E. coli strain (KL188 (thi1, pyrD34, his68, trp95, mt12, xy17, malA1, galK35, rpsL118, λ[R], λ[-]), obtained from B. Bachmann, Coli Genetic Stock Center, Yale University, which was counterselected in a subsequent mating with S. typhimurium LB5010 by omission of histidine, uracil, and thymine from the minimal media supporting growth of LB5010. The lamB[+] F prime factor was moved into attenuated vaccine strains of Salmonella by mating with LB5010, counterselecting the donor strain in a defined media containing all amino acids with the exception of isoleucine, valine, methionine, and leucine.

Haemophilus influenzae Rd strain HDG85 (Deich and Green, J. Bacteriol 170:489-498 (1988)) was used as described in the Examples below.

Bordetella pertussis, strain BP370, discussed below in Example 6, is an FHA[-] isolate of B. pertussis, obtained from Dr. Stanley Falkow, Stanford University, Palo Alto, California, having wild type pertussis toxin and hemolysin production.

(b) Media for Growth of Bacteria

E. coli, S. typhimurium, S. dublin, and S. typhi strains were cultured in L broth. Haemophilus influenzae cells were grown in Brain Heart Infusion broth containing 10 μg/ml hemin and 2 μg/ml NAD. When appropriate, ampicillin was present at 100 micrograms per ml and kanamycin was present at 50 micrograms per ml. pertussis strain BP370 (FHA[-] TOX[+]) was propagated from freezer stocks on semisolid Bordet-Gengou (BG) blood agar (Kimura, A. et al., Infect. Immun. 58:7-16 (1990)). Bacterial mass from agar medium was inoculated into CL medium for liquid cultivation. (Imaizumi et al., Infect. Immun. 41:1138-1143 (1983)).

For inoculation of mice with live Salmonella typhimurium SL3261 derivatives, bacteria were grown in a defined medium consisting of per liter: 6 g $Na_2HPO4$, 3 g $KH_2PO_4$, 0.5 g NaCl, 1 g $NH_4Cl$, adjusted to pH 7.0 and further containing 2 g D-Glucose, 0.2 g $MgSO_4$, 1 mM sodium citrate, 5 g of vitamin-free casamino acids, 5 mg 20 nicotinic acid, 10 mg dihydroxybenzoic acid, 10 mg para-aminobenzoic acid, and 35 mg each phenylalanine, tryptophan, and tyrosine.

Mice were orally inoculated with up to $10^{10}$ bacteria by direct intubation into the stomach with a blunt-ended needle. Bacteria grown as above were centrifuged, washed, and resuspended in 1.5% sodium bicarbonate.

(c) Genetic Methods

Plasmids were transformed into E. coli strains or S. typhimurium LB5010 by the $CaCl_2$ method (Dagert and Ehrlich, Gene 6:23-28 (1979)).

Bacteriophage $P_{22}$ HT int105 was used for generalized transduction of genes from S. typhimurium LB5010. LB5010 was sensitized to phage $P_{22}$ by induction of LPS synthesis by inclusion of D-galactose in the growth media of a log phase culture for one hour prior to phage infection for production of phage lysates. $P_{22}$ phage prepared on LB5010 were mixed at a multiplicity of infection of 1 with SL3261, SL1438, or Ty523 and transductants were selected on suitable medium.

(d) Oligonucleotide Synthesis

Oligonucleotides were synthesized on an Applied Biosystems (Foster City, CA) 380B DNA synthesizer using $\beta$-cyanoethyl phosphoramidite chemistry.

(e) Expression of Genes

LT-B is the non-toxic subunit of enterotoxigenic E. coli labile toxin (LT) and was expressed in E. coli JM103 in a pUC8 vector by cloning a 590 base pair EcoRI-HindIII fragment into EcoRI-HindIII sites of pUC8. The full length Plasmodium berghei CS protein was expressed in a $P_L$ promoter expression vector and consisted of the entire coding sequence plus 8 amino acids (Met-Glu-Ala-Leu-Ile-Leu-Asp-Lys) at the amino terminus (Fig. 6). The first seven amino acids are derived from the expression vector and a linker and the lysine is derived from the DraI site located one codon upstream from the CS protein coding region.

(f) Electrotransfer Methods for Antigen Detection

Heterologous recombinant protein synthesis was detected in E. coli or Salmonella cells by transblotting protein samples separated by polyacrylamide gel electrophoresis onto nitrocellulose membranes, blocking with 0.5% Tween-20 in PBS, followed by antibody binding in 0.1% Tween-20. Bound antibody was detected with horseradish peroxidase-labeled Protein A (Kirkegaard and Perry, Gaithersburg, MD). For detection of LT-B antigen, the primary antibody was goat $\alpha$-LT-B polyclonal reagent partially purified by eluting bound material from a Sepharose 4B protein A affinity column.

(g) Electrotransformation of Haemophilus Influenzae

Haemophilus Influenzae cells were grown in BHIxv medium to mid-log phase (OD600 = 0.3) at 37°C. Cells were pelleted, washed twice with 10 mM Hepes, pH7.0, 20% w/v glycerol and resuspended in 1/100 volume IOmM HEPES pH7.0/20% (v/v) glycerol. Plasmid (pPX1575) DNA was added to ~10$\mu$g/ml and cells were electroporated with a BTX Transfector 100 electroporator. Cell suspension (~50 microliters) were diluted into 1 ml BHIxv media and grown for 2 hours at 37°C to allow expression of the transduced marker. Cultures were then serially diluted in BHI and plated on BHIxv-kanamycin (20 $\mu$g/ml) selective agar and incubated at 37°C.

h) Electrotransformation of Bordetella pertussis.

Starter cultures of BP370 in CL medium were incubated overnight at 35-37°C and then inoculated into 1.3 liters of CL medium and incubated with shaking for 18-24 hours at 35°C. Cultures were harvested by centrifugation at 5000 x g for 30 minutes in a Sorvall GS3 rotor. The cell pellet was resuspended in 200 mls of 0.001 M HEPES buffer, pH 7.2, and subjected to 2 more successive rounds of washing in 0.001 M HEPES buffer. Finally, cells were resuspended in 20 mls 0.001 M HEPES buffer containing 12% glycerol. Cells were frozen and retained at -70°C. During washing operations, cells were maintained at O°C. For electroporation of DNA, an appropriate amount of DNA (up to 10 $\mu$g) was added to 450 $\mu$1 of a thawed cell suspension and mixtures were subjected to an electrical pulse of 33.6 Kilovolts/cm 380 microseconds in duration. Immediately after electroporation, cells were diluted in 1 ml of unsupplemented CL medium and incubated at 37°C for one hour before plating onto BG blood agar plates containing either 25 $\mu$g kanamycin per ml or 50 $\mu$g ampicillin per ml.

i) Southern Blotting of Chromosomal DNA of S. Typhimurium.

A chromosomal DNA was prepared from 5 ml overnight cultures of candidate clones by lysing bacteria in 600 $\mu$1 of TE containing .5% SDS and 100 $\mu$g proteinase K per ml. Cell wall debris and polysaccharides were removed by addition of 1% cetyltrimethyl ammonium bromide (CTAB) in 0.7 M NaCl at 65°C followed

by extraction with chloroform/isoamyl alcohol. Chromosomal DNA was precipitated with isopropyl alcohol, pelleted, dried, and redissolved in TE. Chromosomal DNA was digested with appropriate restriction enzymes and the restricted DNA was subjected to electrophoresis on 0.6% Agarose and transfer to nylon membranes.

DNA probes were prepared from CsCl purified plasmid preparations by labeling by random primed incorporation of digoxigenin-labelled deoxyuridine-triphosphate. Hybridization conditions were developed according to procedures described in Molecular Cloning (Maniatis, T. et al., Cold Spring Harbor, 1982). Detection of the digoxigenin-labelled hybridized DNA was according to the supplier (Boehringer Mannheim).

EXAMPLE I

Construction of Plasmid Vectors Containing Modified Transposition Functions

As shown in Figure 2, pNK1210 was used as a parental plasmid vector in the creation of a construct to insert a DNA sequence (e.g., an expressible gene) stably into the chromosome of gram negative bacteria. pNK1210 contains a tac promoter controlled transposase, the inverted repeats of TnlO and a gene encoding chloramphenicol acetyl transferase. pNK1210 was derived from pNK861 (Way et al., Gene 32:369-379 (1984)) by replacement of the $Kan^R$ determinant with a 1375 bp fragment carrying the $Cm^R$ gene from Tn9. The construct was modified by removing the natural inverted repeats of TnlO and replacing them with an oligonucleotide specifying an inverted repeat sequence comprising the terminal 31 base pairs of the right-end of TnlO flanking a single cloning site (Fig. 3A) to yield plasmid pPX1569. A second oligonucleotide linker (Fig. 3B) was inserted to yield a plasmid with several cloning sites (pPX1572), which was further modified by inserting a $Kan^R$ determinant of Tn5 into the XhoI site of pPX1572. In this configuration, genes inserted into cloning sites flanked by the inverted repeats can transpose in the presence of active transposase.

A mating assay was performed to verify that the 31 5 base-pair synthetic oligonucleotide-derived inverted repeat sequence of pPX1575 acts as a substrate for transposase and yields transposition events. pPX1575 was transformed into F112/C600 which was subsequently mated with E. coli KBTOOl following induction of transposase functions with IPTG. Selecting $Mal^+$ or $Kan^R$ exconjugants of KBTOOl revealed that approximately $10^{-3}$ $Mal^+$ exconjugants were also $Kan^R$ and $Amp^S$ and 100/100 $Kan^R$ exconjugants were $Mal^+$. A similar experiment conducted with F112/C600 transformed with a similar vector lacking the inverted repeat sequences yielded $Mal^+$ but not $Kan^R$ exconjugants. These results indicate that the $Kan^R$ determinant had transposed onto the F factor and the 31 base-pair inverted repeat, as expected, is a substrate for transposase.

EXAMPLE 2

Bacteriophage λ Suicide Vectors for Transposition in Salmonella

Suicide delivery, as used herein, refers to the introduction of a transposon via a DNA construct which cannot replicate in the recipient. ColEI-, p15a-, and pSCIOl-based plasmid vectors replicate freely in S. typhimurium and most other Salmonella species. As such, these vectors are not suitable for suicide delivery of modified transposons in Salmonella. On the other hand, bacteriophage λ will not undergo lytic growth in Salmonella unless nusA, a wild type E. coli host function which is required for λ N protein-dependent antitermination of lambda DNA transcription, and lamB, which is required for λ phage attachment, are present. In addition, λ phage does not lysogenize S. typhimurium because of a requirement for the nusA gene product (Harkki, Mol. Gen. Genet. 209:607-611 (1987)). Thus, λ phage vehicles are not propagated in Salmonella and can be used as suicide delivery vehicles for transposon mutagenesis of this host if the lamB gene product (the outer membrane receptor for phage λ) is provided. As shown below in Table I, a series of bacteriophage λ strains derived from either of the widely studied λgtll 15 strains or λNM816 was tested for ability to form plaques (i.e., undergo lytic growth) on several Salmonella strains which carried the F112dTnlO(cm) conjugative plasmid. Although capable of forming plaques on E. coli strain MC1061, none of the phage was capable of forming plaques on a variety of Salmonella strains. It is not understood why the phages (all of which carry the nin5 deletion) do not form plaques on $lamB^+$ Salmonella (Harkki et al., Mol. Gen. Genet. 209:607-611 (1987)). Modified versions of these bacteriophage carrying transposase recognition sequences and transposase functions can be used as suicide delivery systems.

·

TABLE 1

Efficiency of Bacteriophage λ Plaquing

on Salmonella and E. coli

| | E. Coli MC1061 | S. typhimurium F112cm/ LB5010 | S. typhimurium F112cm/ SL3261 | S. typhi F112cm/ Ty523 |
|---|---|---|---|---|
| λgt11 | $1 \times 10^{10}$ | <20 | <20 | <20 |
| λNM816 | $1 \times 10^{10}$ | <20 | <20 | <20 |
| λPB100[3] | $5 \times 10^{9}$ | <20 | <20 | <20 |
| λPB103[1] | $7 \times 10^{9}$ | <20 | <20 | <20 |
| λPB105[3,4] | 3 x 109 | <20 | <20 | <20 |
| λPB107[1,2] | 1 x 109 | <20 | <20 | <20 |

Notes:

[1] Derivatives of λNM816

[2] Specialized transposing and transducing phage for P. berghei CS protein

[3] Derivatives of λgt11

[4] Specialized transducing phage for LT-B

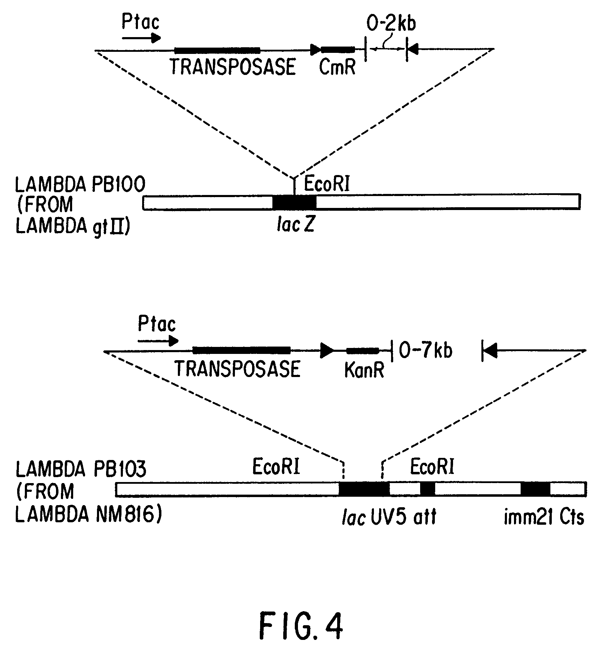

Both λ phages described in Figure 4 were modified to contain a transposase function and TnlO inverted repeats flanking a selectable marker. λPBIOO was constructed by cloning a 5.2 kb EcoRI fragment of DNA from pNK1210, containing a tac promoter controlled transposase gene, the inverted repeats of TnlO and a chloramphenicol acetyl transferase gene into the EcoRI site of λgtll. Cm$^R$ lysogens were selected in E. coli Y1088 in which the transposase is repressible by the laci gene product contained on plasmid pMC9. λPBIOO can accommodate the insertion of up to 2 kb of extraneous DNA.

λPBIO3 was constructed in an analogous fashion by subcloning a 5.2 kb EcoRI fragment of pPX1575 containing the tac promoter controlled transposase, a 31 base pair inverted repeat derived from TnlO flanking a Kan$^R$ determinant from Tn5 and a multiple cloning site, into EcoRI sites of λNM816, resulting in replacement of the lac UV5 stuffer fragment. Kan$^R$ lysogens of MC1061 (pMC9) were selected. λPB103 can accommodate up to 7 kb of extraneous DNA.

Such λ phage vectors, which contain the transposase gene outside the inverted repeats which are the substrate for site specific recombination and transposition, will act not only as temperate transducing phage particles in E. coli, but will catalyze transposition of any DNA (e.g., an expressible gene) contained within the inverted repeats. Since the transposase gene is outside the inverted repeats, the insertion can be stabilized. Because such phage vectors cannot be propagated in Salmonella lamB$^+$ or other lamB$^+$ recipients, either lytically or as lysogens, the phage and consequently the transposase protein is diluted out through the process of cell division. As the concentration of the transposase protein decreases, any transposon which has transposed from the phage to the bacterial chromosome will be stabilized.

A DNA sequence (e.g., an expressible gene) can be introduced between the inverted repeats of bacteriophage vectors such as those shown in Figure 4. One way in which this can be accomplished is by plasmid-phage transposon exchange as shown schematically in Figure 5. A DNA sequence (e.g., an expressible gene) is cloned into a plasmid vehicle (e.g., pPX1575) such that the selectable marker and the expressible gene are flanked by the inverted repeats. In a host cell, lysogenic for a phage of the type depicted in Figure 4, site specific recombination or transposition between the inverted repeats of the plasmid and the lysogenic phage will result in a proportion of the lysogens containing the expressible gene and the selectable marker from the plasmid. The small proportion of phage carrying the exchange event can be selected in E. coli hosts by selecting for lysogens carrying the selectable marker originally associated with the plasmid. The unselected expressible gene is linked with this marker.

EXAMPLE 3

Plasmid/Phage Exchange of an Expressible E. Coli Gene Flanked by Transposase Recognition Sequences

LT-B can be expressed in Salmonella species and has proven to be immunogenic in mice when expressed and administered in live attenuated vaccine strains of Salmonella (Clements et al., Infect. Immun. 53:685-692 (1986)).

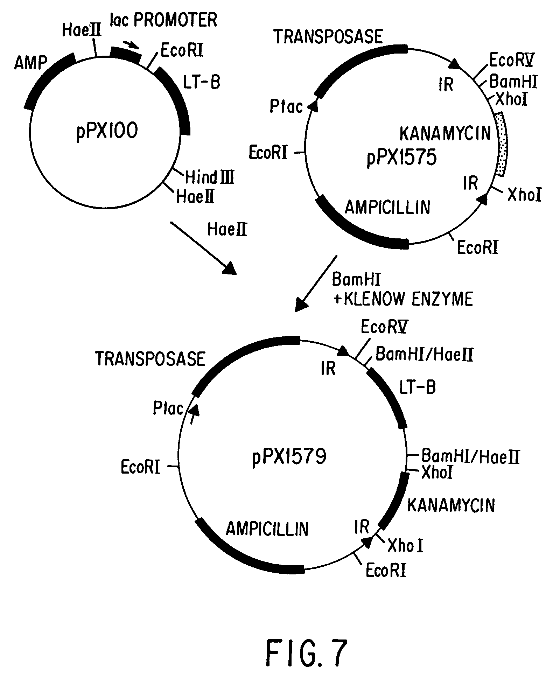

Figure 7 illustrates the construction of a transposable genetic cassette carrying a DNA sequence encoding the 11,000 dalton B subunit of enterotoxigenic E. coli heat-labile toxin (LT-B). The gene specifying the non-toxic subunit was localized on a 600 base pair EcoRI/HindIII fragment in pPX100 in which LT-B expression was controlled by the lac promoter, the sequence of the gene corresponding to the data of Leong et al. (Infect. Immun. 48:73-77 (1985)). An 800 base pair HaeII fragment containing the LT-B coding sequence and the lac promoter was subcloned into the BamHI site of pPX1575, which had been blunt-ended by the action of the Klenow fragment of DNA polymerase I. λPB103 was integrated into JM103 containing the LT-B transposition plasmid pPX 1579. The resulting lysogen was induced to transpose by the addition of IPTG and phage particle production induced by temperature-shock. $Kan^R$, $Cm^S$, lysogens of JM103 were selected.

Approximately 25% of $Kan^R$ transductant clones were also $Cm^R$, suggesting that they were either double lysogens or contained single lysogens harboring both. $Kan^R$, $Cm^S$ clones were considered to have arisen by exchange of genes flanked by the inverted repeats with inverted repeats of the λPB100 lysogen. Although the $Kan^R$ determinant was used to select for putative exchange, all of the $Kan^R$ clones also expressed LT-B by colony blot analysis following IPTG and temperature induction. This result suggests that the unselected gene expression cassette (LT-B) co-transposed as predicted with the drug-resistance marker. One of the resulting $Kan^R$ lysogens was chosen and phage obtained by induction at 42°C. The resultant phage stock (λPB105) was used to infect S. typhimurium and S. typhi. As discussed previously, such a construct serves as a suicide delivery system. The λPB105 construct cannot be propagated, lytically, or as a lysogen, in Salmonella. However, the transposase gene will be expressible and as described in Example 4, the LT-B gene can transpose with the genetic cassette, to the Salmonella chromosome.

EXAMPLE 4

Transposition in Salmonella

a) Selecting Transposition Events in S. typhimurium and S. typhi

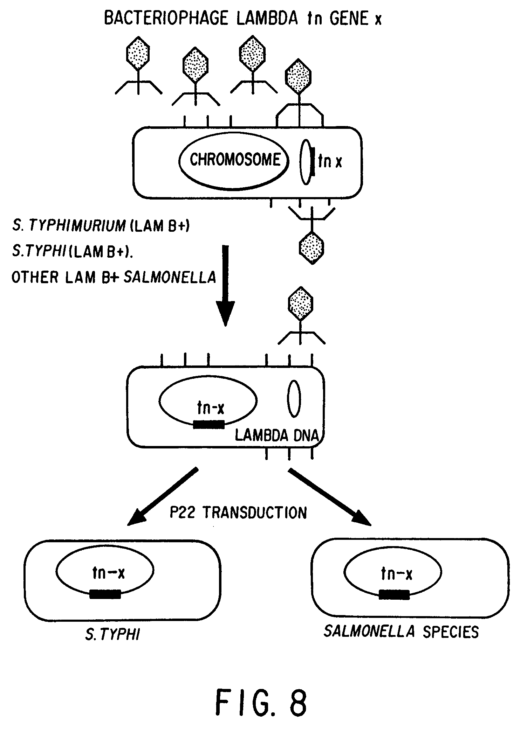

According to the general scheme shown in Fig. 8, several specialized transducing phages were used to transduce S. typhimurium F112dTnlO(cm/LB5010 (lamB+) or S. typhi F112dTnlO(cm)/Ty523 to drug-resistance. Table II shows the results obtained using λPB105. Frequency of transposition was determined by enumerating $Kan^R$ Salmonella colonies following infection with transducing λ phage. λPB105 was mixed with $2 \times 10^8$ recipient Salmonella cells at the indicated m.o.i., which had been grown to midlog phase in the presence of 0.5% maltose to induce the bacteriophage λ receptor. Infected cells were incubated for 1.5 hours at 37°C to allow expression of $Kan^R$ before plating.

As shown in Table II, drug-resistant S. typhimurium were obtained only in the $lamB^+$ but not $lamB^-$ S. typhimurium. For each of the λ phages, drug-resistant clones arose at frequencies of up to $10^{-4}$ at an m.o.i of 0.2 active phage particles in S. typhimurium F112dTnlO(cm)/ LB5010 and with a lower frequency in S. typhi F112dTn(cm)/Ty523.

## TABLE II

### Transposition in Salmonella Using Specialized
### Using Specialized Transposing Phages
### Carrying Heterologous Gene Expression Cassettes

| Donor Bacteriophage | Recipient Salmonella Strain* | | | |
|---|---|---|---|---|
| | F112cm/ | | FM112cm/ | |
| λPB105 | LB5010 | LB5010 | Ty523 | Ty523 |
| m.o.i. = 2 | $4.0 \times 10^{-6}$ | $<1 \times 10^{-8}$ | $5.2 \times 10^{-7}$ | $<1 \times 10^{-8}$ |
| m.o.i. = 2 | $1.0 \times 10^{-4}$ | $<1 \times 10^{-8}$ | $4.0 \times 10^{-7}$ | $<1 \times 10^{-8}$ |
| m.o.i. = 2 | $1.2 \times 10^{-6}$ | $<1 \times 10^{-8}$ | $1.0 \times 10^{-8}$ | $<1 \times 10^{-8}$ |
| no phage | $1.0 \times 10^{-4}$ | $<1 \times 10^{-8}$ | $1.0 \times 10^{-8}$ | $<1 \times 10^{-8}$ |

### b) Characterization of Chromosomal Inserts

Kan$^R$ clones of S. typhimurium LB5010 (lamB$^+$) arising following infection with λPB105 were characterized for expression of the unselected marker and for chromosomal location by Southern blot analysis. Southern blot of KpnI digested chromosomal DNA probed with biotinylated pPX1579 DNA revealed that each of the Kan$^R$ clones had arisen from separate transposition events, since each clone had a single copy of the transposon at random loci in the S. typhimurium chromosome. Further, each of the Kan$^R$ clones expressed the LT-B antigen as determined by Western blot analysis. Expression of the LT-B antigen is controlled by the lac UV5 promoter which is uncontrolled when introduced into Salmonella which are deficient in lactose metabolizing genes. One clone in particular expressed more antigen than the remaining 9 analyzed clones, suggesting that there may be positional effects on the expression of foreign genes integrated at random in the Salmonella chromosome.

### c) Vaccine Strains of Attenuated Salmonella Expressing Integrated Genes

S. typhimurium LT2 derivative LB5010 is a laboratory strain of Salmonella which has some convenient features for manipulation of genes in Salmonella. LB5010 is transformable at high frequency with plasmid DNA and, because of its restriction deficiency, can accept DNA propagated in Escherichia coli. However, it is not itself a vaccine candidate strain. To create vaccine candidates of live attenuated Salmonella, phage P22 HT int105 was propagated on F112dTnIO(cm)/LB5010 derivatives containing modified transposon expressing LT-B and used to transduce several species of aroA deficient Salmonella to Kan$^R$. Transductants of S. typhi Ty523, S. dublin SL1438, and S. typhimurium SL3261 were obtained. Each of the transductants of the different species were demonstrated to express LT-B and further, if the locus at which LT-B was integrated in LB5010 yielded enhanced expression of the antigen, enhanced antigen expression was also observed in the transductants. This indicates the likelihood of transduction of the inserted LT-B expression cassettes, which depends upon homologous recombination, into identical loci in other species of Salmonella.

As an alternative delivery system, the LT-B transposon was integrated into several Salmonella species using temperature-sensitive F' factors. Temperaturesensitive F' factors replicate in a limited number of gram-negative bacterial species, including Salmonella, at low temperatures but fail to replicate at high temperatures. Temperature-sensitive F' factor containing an intact TnIO to supply the transposase function, the lactose operon, and the Kan$^R$ LT-B transposon was isolated in E. coli and then was introduced into S. typhimurium LB5010 by mating from E. coli and selection for Tet$^R$ and lactose utilization at low temperature. The Kan$^R$ LT-B transposon was constructed first in E. coli by transforming an F'$_{ts}$lacTn10 strain with plasmid pPX1579. Transposition of LT-B to the F' factor was induced with IPTG and Kan$^R$, Tet$^R$, Lac$^+$ exconjugants were selected in a mating with E. coli KBTOOI. The F'$_{ts}$lacTn10 carrying the defective LT-B Kan$^R$ transposon was subsequently introduced into S. typhimurium LB5010 by mating. The F' factor in S.

typhimurium LB5010 was then introduced into S. typhi Ty523 (S. dublin SL1438), and S. typhimurium SL3261, by mating selecting for Kan$^R$ Tet$^R$ Lac$^+$ exconjugants and counterselecting against the LB5010 donor strain. LT-B transposition events were selected in each of these strains by selecting Lac$^-$, Kan$^R$, Tet$^S$ colonies following 40 generations of growth in broth lacking drug selection at high temperature (42°C). All of the Kan$^R$ Tet$^S$, Lac$^-$ colonies, which were presumed to have lost the F' factor by segregation, expressed LT-B as determined by colony blot for LT-B antigen. Transposition of the modified transposons from the temperature-sensitive F factor is dependent upon the transposase supplied by the TnIO also located on the F factor.

d) Stability of Transposition

The modified transposons do not retranspose to other loci because the transposase function which is normally encoded within the right inverted repeat of TnIO, are absent. Further, transposition in Salmonella is stable since no ISIO elements (i.e., no constituent transposase genes), which have been detected in numerous E. coli strains, have ever been detected in Salmonella typhimurium (Kleckner et al., Mobile DNA, ASM Publications, Washington, D.C. (1989)). In addition, stable transposition events will result in gene expression which should be inherently stable when compared to plasmid born copies of the same gene, which may be lost in absence of positive selection due to natural segregation of the plasmid or due to selection for loss of the plasmid because of deleterious effects of gene expression.

To test stability of LT-B expressing transposons, one SL3261 isolate, λ3 isolated by P22 transduction of Kan$^R$ segregants from F112dTnIO(cm)/LB5010 (λ3) was grown in L broth lacking kanamycin for 40 generations as shown in Table III. To compare, pPXIOO/SL3261 (a pUC8 plasmid derivative expressing LT-B) and pUC8/SL3261 were also grown without ampicillin selection for 40 generations in LB. In the first example, from cultures of SL3261 (λ3), 100% of the colonies arising on LB agar medium were Kan$^R$ and expressed LT-B antigen; likewise, there was no difference in the number of colonies recoverable by plating directly on LB containing kanamycin or lacking it. On the other hand, only 3% of the colonies from the pPXIOO/SL3261 culture arising on LB medium following 40 generations of growth without drug selection were and by direct plating only 3% of the colonies able to grow on LB agar were recoverable on LB agar containing ampicillin. Further, there was no apparent decrease in gene expression of LT-B in cultures of λ3 relative to pPXIOO/SL3261. This indicates that the transposition event yielded stable expression without selection under culture conditions in an attenuated vaccine strain of Salmonella.

## TABLE III

## Stability of LT-B Expression Plasmid

## versus Chromosomally Integrated LT-B

| Bacterial Strain | % Retention under Culture Conditions after 40 Generations of Passage (% Expressing LT-B) | |
| --- | --- | --- |
| | Defined Medium | Defined Medium + Drug |
| pUC8/SL3261 | 95% (N/A) | 100% |
| pPX100/SL3261 | 3% (3%) | 100% |
| SL3261λ3 | 100% (100%) | 100% |

e) Stability of Gene Expression of Vaccine Strain of Salmonella Containing LT-B Transposon

An isolated transductant of SL3261, an aroA vaccine strain of S. typhimurium, was examined for retention of LT-B expression after immunizing Balb/c mice with a single dose of $10^9$ bacteria per os (orally). As controls, separate groups of mice were orally immunized with either $10^9$ pUC8/SL3261 or $10^9$

pPXIOO/SL3261. Salmonella were cultured from isolated liver, spleen, or lymph node homogenates obtained from immunized mice and the proportion of plasmid-containing organisms or stable transposon-containing organism was determined.

As shown in Table IV, very few Salmonella were obtained from tissue homogenates of mice immunized with either pUC8/SL3261 or pPXIOO/SL3261 and of those only a small percentage were demonstrated to contain plasmid of the original vaccinating dose. Several lymph nodes surrounding the intestine were dissected from each of the vaccinated animals on day 10 or day 15 following oral vaccination of Balb/c mice with $10^9$ bacteria. Lymph node samples were homogenized and duplicate samples plated on LB or LB+ selective drug medium. In addition, the proportion of drug-resistant colonies was determined by replica plating colonies arising on non-selective medium. On the other hand, mice immunized orally with 109 SL3261($\lambda$3) contained $Kan^R$ organisms on days 3, 10, and 15 following vaccination. Further, 100% of the bacterial colonies obtained by plating organ samples on nonselective medium were $Kan^R$ and random testing of 20 $Kan^R$ isolates showed expression of the LT-B antigen. These results demonstrate that integration of a single copy of an expression cassette encoding an antigen can stabilize expression relative to plasmid-borne copies of the same expression cassette in the context of a live vaccine strain of Salmonella.

## TABLE III

### Stability of Integrated Forms of LT-B

### Following Oral Vaccination of Mice

| Vaccine Strain | Animal # | Day 10 | | | Day 15 | | |
|---|---|---|---|---|---|---|---|
| | | Total | $Drug^R$ | % | Total | $Drug^R$ | % |
| pCU8/SL3261 | 1 | 2 | 2 | 100 | 1 | 1 | 100 |
| | 2 | 0 | 0 | - | 0 | 0 | - |
| | 3 | 1 | 1 | 100 | 158 | 3 | 2 |
| pPX100/SL3261 | 1 | 3 | 3 | 100 | 59 | 0 | 0 |
| | 2 | 36 | 0 | 0 | 44 | 0 | 0 |
| | 3 | 360 | 0 | 0 | 0 | 0 | - |
| SL3261$\lambda$3 | 1 | 612 | 612 | 100 | 0 | 0 | - |
| | 2 | 360 | 360 | 100 | 103 | 103 | 100 |
| | 3 | 456 | 456 | 100 | 102 | 102 | 100 |

f) Transposition of Expression Cassettes Containing Alternate Promoters and Gene Regulatory Signals

The method used in the example presented above for stable integration of an LT-B antigen can be generally applied to other proteins whose expression is controllable by various gene regulatory signals which can enhance levels of gene expression. To provide several examples of the utility of this method for creating gene insertions for either protein expression or use in a live vaccine strain of Salmonella, gene expression cassettes containing the strong leftward promoter of bacteriophage $\lambda$ ($P_L$) were incorporated into pPX1575 and $Kan^R$ derivatives of $\lambda$PB104 were obtained by plasmid-phage Tn exchange.

Likewise, as shown in Figure 6, the protective surface antigen (CS protein) from a murine malaria parasite, Plasmodium berghei, was expressed in a $P_L$ expression vector. A series of plasmid constructs was performed in which the entire coding sequence of the P. berghei CS gene was inserted into the $P_L$ promoter expression vector pPX1600. The gene sequence for the P. berghei CS protein is according to Eichinger et al. (Mol. Cell. Biochem. 6:3965-3972 (1986)). The cassette for the expression of the CS gene was contained on a 2.3 kb BamHI restriction fragment which was subcloned into BamHI site of the transposition vector pPX1575. The resulting plasmid was transformed into a host strain lysogenic for $\lambda$PB104. $\lambda$PB104 was

derived from λPB103 by exchange with CmR of PNK1210. Phage particles which had exchanged the plasmid located $Kan^R$ determinant were selected as $Kan^R$, $Cm^S$ lysogens which carried the expressed CS gene. The CS protein incorporated into pPX1575 yielded λPB107.

To obtain host E. coli strains in which both the PL promoter and the lac UV5 promoter are repressed, either plasmid pMc9, which contains the laci gene in a pBR322 plasmid, was transformed into E. coli N99cl[+] or λgtII was lysogenized into JM103 or JM109. Initial plasmid constructs for expression of the P. berghei CS in pPX1575 were recovered in E. coli JM103 (λgtII) to control both expression of transposition functions by the tac promoter and possible deleterious effects of the PL-promoted expression of the CS protein in E. coli. pPX1575 derivatives harboring expression cassettes of the CS protein were lysogenized with λPB103 to create a strain doubly lysogenic for λgtII and λPB103 for the exchange reaction. λPB103 lysogens of JM103 would not be suitable hosts to repress the expression of genes controlled by the PL promoter because λPB103 contains the phage 21 immunity region. The double lysogens were induced for phage functions by temperature shifts of candidate cultures and $Kan^R$ lysogens were selected in N99cl[+] - (pMC9). Lysogens which were $Kan^R$ and $Cm^S$ were retained as candidates and were presumed to have arisen by exchange of plasmid-born $P_L$ expression cassettes and selectable markers flanked by synthetic inverted repeats with the inverted repeat region of λPB103.

g) Bacteriophage λ Cloning Vehicles for Directly Generating Specialized Transposing Phage Lines

Derivatives of λgtII and λNM816 are created which are suitable cloning vectors for the generation of λ phage which can be used directly for insertion of expressed genes into chromosomes of other organisms using standard splicing techniques rather than lysogenplasmid transposon exchange. This is accomplished by subcloning the lacα region of pUC18 into the BamHI site of pPX1575, followed by cloning a linker into the XbaI site of the PUC18 polylinker region. Such linkers may encode the in-frame readthrough of the lacα peptide and, for example, any or all of the NotI, SfiI, and SpeI restriction enzyme recognition sequences which are not otherwise present in bacteriophage λ. The modified pUC18 polylinker region may be subcloned as a 400 bp HaeII fragment into the filled out BamHI site of pPX1575. By exchange of this modified pUC18 region into λPBIOO (a λgtII derivative) or λPB103 (a λNM816 derivative), λ cloning vehicles are generated such that antigens or proteins can be cloned into any of the three unique cloning sites using suitable adaptors or linkers by screening either for expression of the protein or for insertion of cloned DNA by interruption of the lacα function in a JM103 or JM109 genetic background, provided that the hfl mutation allowing for high frequency lysogenization is also present. The resultant phages can be used directly for constructing unique live vaccine strains without the necessity of separate expression steps.

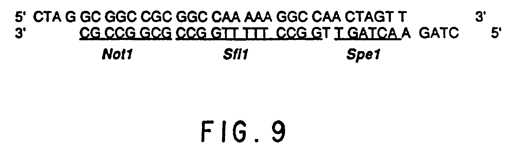

A bacteriophage lambda similar to the ones described above was isolated and characterized. Bacteriophage λPB113 was isolated by plasmid-lysogen exchange as described above using pPX1591 as the plasmid vehicle and λPB104 as the lysogen. Bacteriophage λPB104 was derived from λPB103 by exchange of kanamycin-resistance with chloramphenicol-resistance. Thus, λPB104 is a derivative of λNM816 containing the TN10 transposase controlled by the tac promoter located outside the inverted repeats of TN10 which flank the chloramphenicol acetyl transferase gene. Plasmid pPX1591 was isolated as follows. A 400 base pair HaeII fragment isolated from plasmid pUC18, blunt-ended with T4 polymerase, was cloned into the klenow-enzyme treated BamH1 site of pPx1575. This intermediate vector, termed pPX1577, contained the lacα peptide and the multiple cloning site of pUC18, a kanamycin-resistance determinant, flanked by the synthetic 31 base-pair inverted repeats of Tn10. This plasmid was digested with XbaI and the double stranded oligonucleotide linker, shown in Figure 9, was inserted.

This oligonucleotide, when inserted in the orientation shown in Figure 9 in the XbaI site of pPx1577, yielded a plasmid which contained a functionally active lacα peptide which complemented the lacZ(M15) deletion of E. coli JM103. Colonies that had the oligonucleotide linker in the opposite orientation yielded a non-functional lacα peptide. The resulting plasmid, termed pPX1591, contained unique NotI and SfiI restriction enzyme sites and in addition regenerated a unique XbaI site at the 3' end of the linker. DNA sequencing using standard M13 primers revealed that the inserted oligonucleotide was present as indicated. To isolate λPB113, E. coli JM103 containing pPX1591 was infected with choramphenicol-resistant λPB104 at 30°C in log-phase broth cultures induced for the lambda receptor by inclusion of 0.2% maltose. When cultures had reached late-log phase, they were induced to lyse by temperature-shock at 42°C. The resulting phage lysate was used to infect a fresh culture of JM103, which was subsequently selected for kanamycin-resistant lysogens which formed blue colonies on LB agar medium containing 200 micrograms X-gal per ml. A lysogen which transduced kanamycin-resistance at high frequency was termed λPB113. DNA isolated from this bacteriophage contains NotI and SfiI sites which are unique to bacteriophage lambda. Thus, λPB113 has properties which allow cloning of DNA fragments as large as 7 kilobases into the unique NotI and SfiI sites, which can be screened in kanamycin-resistant lysogens as white colonies on medium containing X-gal using an E. coli host carrying a lacα deletion. Further, the lac promoter can promote expression of the cloned inserted DNA if the inserted DNA is in the correct reading frame with respect to the lacα peptide and has the appropriate initiation signals. The phage have the additional property that the cloned DNA inserts are essentially transposable elements such that expressed DNA inserts can be examined by stable integration into a bacterial host chromosome.

An E. coli JM103 lysogen carrying bacteriophage λPB113 in has been deposited with the National Regional Research Laboratory (NRRL) culture collection and has been given the Accession No. B-18871.

## EXAMPLE 5

### Transposition in Haemophilus influenzae

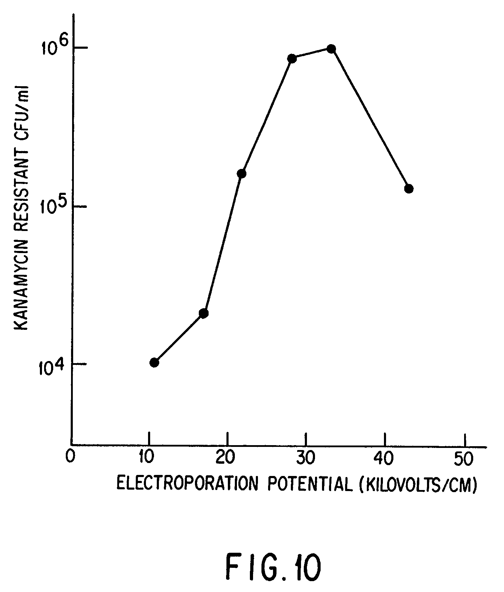

Transposition of Kan$^R$ from pPX1575 was tested in H. influenzae Rd strain HDG85. This plasmid should act as a suicide delivery system in H. influenzae, as colEI derived plasmids have been shown not to replicate in H. influenzae cells (Danner and Pifer, Gene 18:101-105 (1982)). Plasmid DNA was introduced into H. influenzae cells via electroporation and Kan$^R$ recombinants were scored. As summarized in Figure 10, pPX1575 (~10 micrograms) was added to H. influenzae HGD85 cells, which were subjected to electroporation of the voltages indicated. The total number of Kan$^R$ microcolonies per ml of electroporation mixture is plotted verses the electroporation voltage. Under the conditions used, Kan$^R$ derivatives of HDG85 were obtained with optimal transduction to Kan$^R$ at ~30 kilovolts/cm.

When electroporation mixtures of H. influenzae and pPX1575 were plated on selective media, a large number of microcolonies were visible after 24 hours. However, most of these colonies failed to grow further, and did not regrow when subcultured on kanamycin plates. However, after 72 hours, 1-2% of these microcolonies developed into apparently normal H. influenzae colonies. Cells taken from these colonies regrew under kanamycin selection. No microcolonies were observed and no stable Kan$^R$ recombinants were obtained when cells were electroporated with vector DNA, or when cells and pPX1575 were mixed but not electroporated.

## EXAMPLE 6

### Transposition in Bordetella Pertussis

Transposition of the Kan$^R$ determinant contained within the 31 base pair inverted repeat sequence of pPX1575 was demonstrated in B. pertussis strain BP370. Plasmid DNA was introduced into BP370 by electroporation. Transposition events were selected by plating electroporated cells on selective medium containing kanamycin. Kan$^R$ microcolonies arose with a frequency of greater than 1 per $10^5$ viable cells and were visible on the selection plates within 48 hours post plating. These colonies were grown further and individually tested for regrowth on kanamycin plates and plates containing ampicillin to check for the inheritance of the plasmid vehicle. Of 75 Kan$^R$ hemolyticcolonies tested, none was also Amp$^R$. By the same token, electroporated cells were plated on medium containing ampicillin to examine the transient inheritance of the plasmid vehicle. The number of Kan$^R$ colonies arising on selection plates was a 1000 times greater than the number of colonies arising on ampicillin-containing medium. The low frequency of Amp$^R$ transformants results from the unstable inheritance of the plasmid vehicle in the recipient cells: approximately 15% of the Amp$^R$ clones were simultaneously Kan$^R$, suggesting plasmid rearrangements in the recipient cells as a consequence of expression of the TnIO transposase. These data indicate that high frequency transformation of B. pertussis with DNA unable to replicate stably within the recipient cells but having selectable genetic material contained within 31 base pairs of TnIO inverted repeat DNA results from specific transposition.

It is possible using the cloning vector pPX1575, or derivatives of it suitably modified to contain additional unique restriction enzyme sites and other selectable markers described above, to randomly insert modified pertussis toxin DNA directly into the chromosome of B. pertussis. In an ideal application of this idea, specifically engineered deletion, point, or insertion mutations in the SI subunit of the pertussis toxin operon, designed to genetically reduce to absolute minimum the toxic activity of pertussis toxin, yet retaining ability to assemble into holotoxin, can be cloned along with its native promoter into appropriate cloning sites of the transposition vector. By electroporating such DNA into host B. pertussis essentially deleted of all host toxin operon sequences and selecting for the co-inherited marker, one or more random insertions of the modified operon can be obtained.

Equivalents

Those skilled in the art will recognize, or be able to ascertain using no more than routine experimentation, many equivalents to the specific embodiments of the invention described herein. Such equivalents are intended to be encompassed by the following claims.


**Sequence ID No.:  1**

**Sequence Type:  DNA**

**Sequence Length:  72 Base Pairs**

**Strandedness:  Double**

**Topology:  Linear**

**Properties:  HindIII, XhoI Restriction Sites**


AGCTCTGATG AATCCCCTAA TGATTTGGGT AAAAACTCGA GTTTTTACCA          50

AAATCATTAG GGGATTCATC AG                                        72


**(Figure 3A)**


**Sequence ID No.:  2**

**Sequence Type:  DNA**

**Sequence Length:  47 Base Pairs**

**Strandedness:  Double**

**Topology:  Linear**

**Properties:  XhoI, BamHI, EcoRV Restriction Sites**


ATTTTGGTAA AACTCGAGGA TCCGATATCG TCGAGTTTTA CCAAAAT          47


**(Figure 3B)**

**Sequence ID No.:  3**

**Sequence Type:  DNA**

**Sequence Length:  33 Base Pairs**

**Strandedness:  Double**

**Topology:  Linear**

**Properties:  NotI, SfiI Restriction Sites**


**CTAGGCGGCC GCGGCCAAAA AGGCCAACTT AGT**                    **33**


**(Figure 9)**


## Claims

1.  A DNA construct for introducing a DNA sequence into the constituent DNA of a prokaryotic cell, the construct comprising a gene encoding a transposase protein linked in cis to a transposable cassette, the cassette comprising a pair of transposase recognition sites flanking a cloning site for insertion of the DNA sequence, but not flanking the gene encoding the transposase protein.

2.  A DNA construct of Claim 1, wherein the transposable cassette further comprises a selectable marker and the DNA sequence, both the selectable marker and the DNA sequence being flanked by the transposase recognition sites.

3.  A DNA construct of Claim 1 or 2, wherein the DNA sequence is an expressible gene from a source selected from the group consisting of bacterial, viral, parasite, fungal and mammalian sources.

4.  A DNA construct of Claims 1-3, wherein the pair of transposase recognition sites are identical or substantially homologous and functionally equivalent to the transposase recognition sequences of a prokaryotic transposon.

5.  A DNA construct of Claims 1-4, wherein the prokaryotic cell is a bacterium and the DNA sequence encodes an antigen capable of stimulating an immune response.

6.  A DNA construct of Claim 5, wherein the bacterium is an attenuated enteroinvasive bacterium, selected from the group consisting of Escherichia coli, Yersinia enterocolitica, Shigella flexneri, S. dysenteriae, Campylobacterium jejuni, Vibrio cholerae, Bacille Calmette-Guerin, avirulent mutants of Mycobacterium bovis, Salmonella typhi, Salmonella dublin, Salmonella typhimurium, Salmonella gallinarium, Salmonella paratyphi and Salmonella cholerae suis.

7.  An episome for introducing DNA into the constituent DNA of a prokaryotic cell, the episome comprising:
    a) a gene encoding a transposase protein;
    b) a transposable cassette linked in cis to the gene encoding the transposase protein, the transposable cassette comprising a pair of inverted repeats flanking a multiple cloning site and a selectable marker, but not flanking the gene encoding the transposase protein; and
    c) a replicon having an origin of replication which is substantially homologous and functionally equivalent to a prokaryotic origin of replication.

**8.** A bacteriophage derivative for introducing DNA into the constituent DNA of a prokaryotic cell, the bacteriophage derivative comprising:

a) a gene encoding a transposase protein;

b) a transposable cassette linked in cis to the gene encoding the transposase protein, the transposable cassette comprising a pair of inverted repeats flanking a multiple cloning site and a selectable marker, but not flanking the gene encoding the transposase protein; and

c) an origin of replication which is substantially homologous and functionally equivalent to a bacteriophage origin of replication.

**9.** A live vaccine comprising an attenuated bacterium having incorporated in its constituent DNA a transposable cassette containing a gene encoding an antigen of interest.

**10.** A live vaccine of Claim 9, for administration to humans wherein the bacterium is selected from the group consisting of Escherichia coli, Yersinia enterocolitica, Shigella flexneri, Shigella dysenteriae, Campylobacterium jejuni, Vibrio cholerae, Bacille Calmette-Guerin and avirulent mutants of Mycobacterium bovis, Salmonella typhi, Salmonella dublin, Salmonella typhimurium, Salmonella gallinarium, Salmonella paratyphi and Salmonella cholerae suis.

**11.** A method for introducing a DNA sequence into the constituent DNA of a prokaryotic cell comprising the steps of:

a) introducing a DNA construct into the prokaryotic cell under conditions appropriate for transposition, the DNA construct comprising a gene encoding a transposase protein linked in cis to a transposable cassette, the cassette having a pair of transposase recognition sites flanking the DNA sequence; and

b) identifying recipient cells having the DNA sequence inserted into the constituent DNA.

**12.** A method for introducing a DNA sequence into the constituent DNA of a prokaryotic cell at a predetermined genetic locus, the prokaryotic cell containing a resident transposon at the predetermined genetic locus, the method comprising:

a) introducing a DNA construct into the prokaryotic cell, the DNA construct comprising:

i) a transposable genetic cassette having a pair of transposase recognition sites flanking a selectable marker and the DNA sequence;

ii) a gene encoding a transposase protein linked in cis to the transposase cassette, the encoded protein recognizing both the vector born transposase recognition sites, and the transposase recognition sites of the resident transposon; and

b) identifying prokaryotic cells having the DNA sequence inserted into the constituent DNA of the prokaryotic cell at the predetermined genetic locus.

FIG. 1

INSERTED DNA SEQUENCE

SELECTABLE MARKER

TRANSPOSASE

TRS    CS   TRS

TRS—TRANSPOSASE RECOGNITION SEQUENCE
CS —CLONING SITE

FIG. 2

EP 0 485 701 A1

Hind111                                                            XhoI

AGCT CTGATGAATCCCCTAATGAT T T TGGGTAAAAAC TCGAGT T T T TACCAAAATCAT TAGGGGAT TCATCAG

GACTACTTAGGGGATTACTAAAACCCAT T T T TGAGCT CAAAAATGG T T T TAGTAATCCCCTAAGTAGTCTCGA

Hind111

## FIG. 3A

Xhol          EcoRV    BamH1
TCGA CGATATCGGATCC
               GCTATAGCCTAGG TCGA
                                 Xhol

↓

insertion of linker into pPX1572

..................AT T T TGGTAAAAC TCGAGGATCC GATATCG TCGAGTT TTACCAAAAT...................................
..................TA AA ACCATT TTG AGCT CCTAGGC TATAGCAGCTCAAAATGGTT TTA...................................
              *Xhol   BamH1   EcoRV*

FIG. 3B

FIG. 4

FIG. 5

FIG. 6A

FIG. 6B

AMP

pPXI587

IR

IR

BamHI

Kan R  BamHI

LAMBDA PBI04
(FROM LAMBDA PBI03
BY EXCHANGE)

Cm R

TRANSPOSASE

IR

IR

SELECTION FOR
KANAMYCIN-RESISTANT,
CHLORAMPHENICOL-SENSITIVE
LYSOGENS

LAMBDA PBI04
(FROM LAMBDA Pbl03
BY EXCHANGE)

Kan R

TRANSPOSASE

IR  P. BERGHEI
CS GENE

IR

LAMBDA PBI07: PL P. BERGHEI TRANSPOSITION VECTOR

# FIG. 6C

FIG. 7

FIG. 8

```
5' CTA G GC GGC CGC GGC CAA AAA GGC CAA CTAGT T          3'
3'        CG CCG GCG CCG GTT TTT CCG GT T GATCA A  GATC      5'
         Not1              Sfi1              Spe1
```

# FIG. 9

FIG. 10

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | WO-A-8 801 646 (ALLELIX INC.) 10 March 1988<br>* page 34, paragraph 2 - page 36, paragraph 2 *<br>* page 57, paragraph 3 - page 58, paragraph 2 *<br>* figure 11 *<br>--- | 1,7 | C12N15/63<br>C12N15/90<br>A61K39/00<br>C12N15/70<br>C12N15/74 |
| P,X | CHEMICAL ABSTRACTS, vol. 114, no. 1,<br>7 January 1991, Columbus, Ohio, US;<br>abstract no. 1712T,<br>M. HERRERO ET AL.: 'Transposon vectors containing non-antibiotic resistance selection markers for cloning and stable chromosomal insertion of foreign genes in Gram-negative bacteria'<br>page 182 ;column R ;<br>* abstract *<br>& JOURNAL OF BACTERIOLOGY<br>vol. 172, no. 11, November 1990, BALTIMORE, US<br>pages 6557 - 6567;<br>--- | 1-5,7 | //<br>C12N15/30<br>C12N15/31 |
| A | GENE.<br>vol. 51, 1987, AMSTERDAM NL<br>pages 91 - 96;<br>M. G. OBUKOWICZ: 'IS50L as a non-self transposable vector used to integrate the Bacillus thuringiensis delta-endotoxin gene into the chromosome of root-colonizing pseudomonads'<br>* abstract *<br>* page 93, right column, paragraph 2 *<br><br>----- | 1-4,7 | **TECHNICAL FIELDS SEARCHED (Int. Cl.5 )**<br><br>C12N |

The present search report has been drawn up for all claims

| Place of search | Date of completion of the search | Examiner |
|---|---|---|
| THE HAGUE | 17 DECEMBER 1991 | THIELE U.H.-C.H. |